# EUROPÄISCHE PATENTSCHRIFT

(11) **EP 0 606 058 B1**
(45) Veröffentlichungstag und Bekanntmachung des Hinweises auf die Patenterteilung: **05.11.1997**
(21) Anmeldenummer: 94100015.0
(22) Anmeldetag: 03.01.1994
(51) Int. Cl.: C07C 69/65, C07C 67/343, C07C 245/20, C07C 57/60

(54) **Halogenierte Zimtsäuren und deren Ester, sowie Verfahren zu ihrer Herstellung und halogenierte Aryldiazoniumsalze**
Halogenated cinnamic acids and their esters as well as processes for their preparation and halogenated aryldiazonium salts
Acides cinnamiques halogénés et leurs esters ainsi que des procédés pour leur préparation et sels aryldiazonium halogénés

(30) Priorität: 07.01.1993 DE 4300195
(43) Veröffentlichungstag der Anmeldung: 13.07.1994
(73) Patentinhaber: HOECHST AKTIENGESELLSCHAFT, 65929 Frankfurt am Main (DE)
(72) Erfinder: Beller, Matthias, Dr., D-65527 Niedernhausen (DE); Fischer, Hartmut, D-65719 Hofheim (DE); Weisse, Laurent, Dr., D-61440 Oberursel (DE); Forstinger, Klaus, Dr., D-65451 Kelsterbach (DE); Pfirmann, Ralf, Dr., D-64347 Griesheim (DE); Strutz, Heinz, Dr., D-61250 Usingen (DE)

(56) Entgegenhaltungen:
- EP-A- 0 509 426
- DE-A- 2 630 727
- US-A- 3 345 263
- US-A- 4 346 248
- CHEMICAL ABSTRACTS, vol. 58, no. 3, 1963, Columbus, Ohio, US; abstract no. 2389g, & ZH. OBSHCH. KHIM., Bd.32, 1962 Seiten 849 - 853 G.G. YAKOBSON ET AL
- CHEMICAL ABSTRACTS, vol. 51, no. 12, 1957, Columbus, Ohio, US; abstract no. 8667a, & ACTA CHIM. ACAD. SCI. HUNG., Bd.10, 1956 Seiten 227 - 232 A. PAVLATH ET AL
- CHEMICAL ABSTRACTS, vol. 74, no. 25, 1971, Columbus, Ohio, US; abstract no. 139822r, & BULL. MUS. NAT. HIST. NATUR., 1971, PARIS Seiten 799 - 812 D. MOLHO ET AL
- CHEMICAL ABSTRACTS, vol. 95, no. 5, 1981, Columbus, Ohio, US; abstract no. 43172m, & REGISTRY (Datenbank, STN) RN: 78060-26-1 & CS-A-185 059 (M. PROTIVA ET AL)
- TETRAHEDRON, Bd.37, Nr.1, 1981 Seiten 31 - 36 K. KIKUKAWA ET AL

## Beschreibung

Die vorliegende Erfindung betrifft neue halogenierte Zimtsäuren und deren Ester, sowie Verfahren zu ihrer Herstellung und hierfür benötigte neue halogenierte Aryldiazoniumverbindungen.

Als halogenierte Aryldiazoniumverbindungen sind 2,4,5,-Trifluorphenyldiazoniumtetrafluoroborat (Chem. Abstr. 58, 2389 g (1963)), 2,4,5-Tribromphenyldiazoniumtetrafluoroborat (Chem. Abstr. 51, 8667 a (1957)) und 2,5-Dichlor-4-brombenzoldiazoniumchlorid (DE-A-2 630 727) bekannt.

Substituierte Zimtsäuren und Zimtsäureester haben technische Bedeutung. Sie finden als ultraviolettes Licht absorbierende Stoffe in Cremen, Salben und Ölen Anwendung. Neben ihrer Verwendung als Lichtschutzmittel im Kosmetiksektor dienen substituierte Zimtsäuren und ihre Ester als Riechstoffe und als Antioxidantien (Literaturangaben DE 3 139 994, DE 3 012 535, EP 484 122, US 4 970 332, JP 04129790). Darüber hinaus stellen sie einerseits wertvolle Ausgangstoffe und Zwischenprodukte für die Herstellung von Herbiziden, Fungiziden und pharmazeutisch wirksamen Substanzen dar und dienen andererseits selbst als Wirkstoffe, beispielsweise als Lipoxygenaseinhibitoren oder Aldolasereduktaseinhibitoren (JP 04193890, JP 04208211). Die vorstehenden Anwendungsgebiete treffen auch für die neuen halogenierten Zimtsäuren und halogenierten Zimtsäureester zu.

Wegen der allgemeinen Bedeutung und vielseitigen Verwendbarkeit dieser Stoffklasse stellt es eine lohnende Aufgabe dar, neue Verbindungen aus dieser Gruppe von Stoffen bereitzustellen, um das Spektrum ihrer Anwendungsmöglichkeiten nicht nur zu ergänzen, sondern auch durch eine Nuancierung stofflicher Eigenschaften zu bereichern und zu erweitern.

Diese Aufgabe wird gelöst durch Verbindungen der Formel 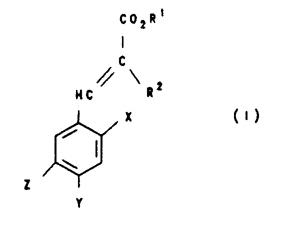 worin R¹ und R² gleich oder verschieden sind und für Wasserstoff oder einen gegebenenfalls Sauerstoff, Stickstoff oder Halogen enthaltenden Alkylrest mit 1 bis 18 Kohlenstoffatomen stehen, die Reste X, Y und Z gleich sind und für ein Fluor-, Brom- oder Jodatom stehen oder, falls zwei der Reste X, Y oder Z gleich oder alle Reste X, Y, Z voneinander verschieden sind, X, Y und Z ein Fluor-, Chlor-, Brom- oder Jodatom bedeuten.

Als Alkylreste kommen generell cyclische, geradkettige oder einfach oder mehrfach verzweigte Alkylreste, die gesättigt oder einfach oder mehrfach ungesättigt sind und gegebenenfalls ein oder mehrere Heteroatome wie Sauerstoff, Stickstoff oder ein Halogen enthalten, in Betracht.
Beispiele hierfür sind die Methyl-, Ethyl-, n-Propyl-, i-Propyl, n-Butyl-, t-Butyl, i-Butyl-, n-Pentyl-, i-Pentyl-, n-Hexyl-, i-Hexyl-, n-Heptyl-, i-Heptyl-, n-Octyl-, i-Octyl-, 2-Ethylhexyl-, Nonyl-, Decyl-, Undecyl-, Dodecyl- Tridecyl-, Tetradecyl-, Hexadecyl-, Octadecyl-, 2-Chlorethyl-, 2-Bromethyl-, 2-Hydroxyethyl-, 2-Hydroxypropyl-, 3-Hydroxypropyl-, 2-Methoxyethyl, 2-Ethoxyethyl-, 4-Ethoxybutyl-, 2-Propoxyethyl-, 2-Butoxyethyl-, 3-Methoxypropyl-, 3-Ethoxypropyl-, 4-Methoxybutyl-, 3-Ethoxybutyl-, 3-Hydroxypropyl-, 4-Hydroxybutyl-, 3-Chlorpropyl-, 2-Chlorpropyl-, 2-Acetoxyethyl, 3-Acetoxypropyl-, 4-Acetoxybutyl-, Cyclopropyl-, Cyclobutyl-, Cyclopentyl-, Cyclohexyl-, Methylcyclohexyl-, Cycloheptyl-, Cyclooctyl-, Cyclodecyl-, Cyclododecyl-, 2,3-Epoxypropyl-, 2-Aminoethyl-, 2-Aminopropyl-, 3-Aminopropyl-, 4-Aminobutyl-, 2-Acetamidoethyl-, 3-Acetamidpropyl-, 4-Acetamidobutyl-, 2-Furyl-, 3-Furyl-, 2-Ethenyl-, 2-Propenyl-, 2-Butenyl-, 3-Butenyl-, 2-Hexenyl-, 3-Hexenyl-, Cyclopentenyl- und Cyclohexenyl-Gruppe.

R¹ steht neben Wasserstoff für einen gegebenenfalls Sauerstoff, Stickstoff oder Halogen, insbesondere Chlor, enthaltenden Alkylrest mit 1 bis 18, insbesondere 1 bis 12 Kohlenstoffatomen. Hierunter fallen gegebenenfalls durch eine Alkoxygruppe oder Acyloxygruppe substituierte geradkettige oder verzweigte Alkylreste mit 1 bis 18, insbesondere 1 bis 12 Kohlenstoffatomen. Die Alkoxygruppen oder Acyloxygruppen nehmen eine beliebige Position ein. R¹ steht insbesondere für Wasserstoff, eine Methyl-, Ethyl-, n-Propyl-, i-Propyl-, n-Butyl-, i-Butyl-, t-Butyl-, n-Hexyl-, i-Hexyl-, n-Octyl, i-Octyl-, 2-Ethylhexyl-, Decyl- oder Dodecyl-Gruppe.

Eine gewisse Bedeutung kommt den freien Zimtsäuren der Formel (I), worin R¹ für H steht, zu, da sie selbst als Zwischenprodukte für die oben angesprochenen Anwendungen interessant sind und sich durch Umsetzung mit Alkoholen in die entsprechenden Ester umwandeln lassen und den Weg zu einer Vielzahl von Estern eröffnen.
Unter Alkylrest werden auch Reste, die für eine -(CH₂)ₙ-OR³ Gruppe, worin n = 2 bis 6 ist und R³ für einen Alkylrest mit 1 bis 4 Kohlenstoffatomen stehen, oder eine -(CH₂-CH₂-O)ₙ-R³ Gruppe, worin n = 2 bis 4 ist und R³ für einen Alkylrest mit 1 bis 4 Kohlenstoffatome steht, verstanden. Dies betrifft sowohl R¹ als auch R², insbesondere R¹.

Interessant sind die Verbindungen der Formel (I), worin R² für Wasserstoff oder eine Methylgruppe steht. Sie lassen sich einfach und ökonomisch herstellen.

Für den Fall, daß X, Y und Z gleich sind, stehen diese Reste jeweils für ein Fluor-, Brom- oder Jodatom. Falls zwei der drei Reste X, Y und Z einander gleich sind, repräsentieren X, Y oder Z ein Fluor-, Chlor-, Brom- oder Jodatom, insbesondere ein Fluor-, Chlor- oder Bromatom, wobei die einander gleichen Reste insbesondere für ein Fluor- oder Chloratom stehen. Sind alle Reste X, Y, Z voneinander verschieden, so bedeuten X, Y und Z ein Fluor-, Chlor-, Brom-oder Jodatom, insbesondere ein Fluor-, Chlor- oder Bromatom.

Hervorzuheben sind Verbindungen der Formel (I), worin die beiden gleichen Reste X und Y für ein Bromatom und Z für ein Fluoratom stehen oder die beiden gleichen Reste Y und Z jeweils für ein Fluoratom und X für ein Bromatom stehen. Daneben sind auch Verbindungen von Bedeutung, in denen X ein Bromatom, Y ein Chloratom und Z ein Fluoratom darstellen.

Von besonderer Bedeutung sind Verbindungen der Formel (I), worin R¹ und R² gleich oder verschieden sind und für Wasserstoff oder einen gegebenenfalls Sauerstoff, Stickstoff oder Halogen enthaltenden Alkylrest mit 1 bis 18, insbesondere 1 bis 12 Kohlenstoffatomen, X für ein Chloratom und Y und Z jeweils für ein Fluoratom stehen. Besonders zu erwähnen sind Verbindungen dieser Art, in denen R¹ für Wasserstoff oder einen Alkylrest, insbesondere einen gesättigten Alkylrest mit 1 bis 12 Kohlenstoffatomen und R² für Wasserstoff oder eine Methylgruppe, insbesondere Wasserstoff stehen.
Beispiele hierfür sind der Methyl-, Ethyl-, n-Propyl-, i-Propyl-, n-Butyl-, i-Butyl-, t-Butyl-, Pentyl-, Hexyl-, Octyl-, 2-Ethylhexyl-, Decyl-, Dodecyl-, 2-Chlorethyl-, 2-Aminoethyl-, 2-Acetamidoethyl-, 2-Acetoxyethyl- und 3-Acetoxypropyl-Ester der 2-Chlor-4,5-difluorzimtsäure.
Bevorzugt sind der Methyl-, Ethyl-, n-Propyl-, i-Propyl-, n-Butyl-, i-Butyl, t-Butyl-, Hexyl-, Octyl- und 2-Ethylhexyl-Ester der 2-Chlor-4,5-difluorzimtsäure.

Ebenfalls von besonderer Bedeutung sind Verbindungen der Formel (I), worin R¹ und R² gleich oder verschieden sind und für Wasserstoff oder einen gegebenenfalls Sauerstoff, Stickstoff oder Halogen enthaltenden Alkylrest mit 1 bis 18, insbesondere 1 bis 12 Kohlenstoffatomen und X, Y und Z jeweils für ein Fluoratom stehen. Besonders zu erwähnen sind Verbindungen dieser Art, in denen R¹ für Wasserstoff oder einen Alkylrest, insbesondere einen gesättigten Alkylrest mit 1 bis 12 Kohlenstoffatomen und R² für Wasserstoff oder eine Methylgruppe, insbesondere Wasserstoff stehen.
Beispiele hierfür sind der Methyl-, Ethyl-, n-Propyl-, i-Propyl-, n-Butyl-, i-Butyl-, t-Butyl-, Pentyl-, Hexyl-, Octyl-, 2-Ethylenhexyl-, Decyl-, Dodecyl-, 2-Chlorethyl-, 2-Aminoethyl-, 2-Acetamidoethyl-, 2-Acetoxyethyl- und 3-Acetoxypropyl-Ester der 2,4,5-Trifluorzimtsäure.
Bevorzugt sind der Methyl-, Ethyl-, n-Propyl-, i-Propyl-, n-Butyl-, i-Butyl-, t-Butyl-, Hexyl-, Octyl- und 2-Ethylhexyl-Ester der 2,4,5-Trifluorzimtsäure.

Gleichfalls von besonderer Bedeutung sind Verbindungen der Formel (I), worin R¹ und R² gleich oder verschieden sind und für Wasserstoff oder einen gegebenenfalls Sauerstoff, Stickstoff oder Halogen enthaltenden Alkylrest mit 1 bis 18, insbesondere 1 bis 12 Kohlenstoffatomen, X und Y jeweils für ein Chloratom und Z für ein Fluoratom stehen. Besonders zu erwähnen sind Verbindungen dieser Art, in denen R¹ für Wasserstoff oder einen Alkylrest, insbesondere einen gesättigten Alkylrest mit 1 bis 12 Kohlenstoffatomen und R² für Wasserstoff oder eine Methylgruppe, insbesondere Wasserstoff stehen. Beispiele hierfür sind der Methyl-, Ethyl-, n-Propyl-, i-Propyl-, n-Butyl-, i-Butyl-, t-Butyl-, Pentyl-, Hexyl-, Octyl-, 2-Ethylenhexyl-, Decyl-, Dodecyl-, 2-Chlorethyl-, 2-Aminoethyl-, 2-Acetamidoethyl-, 2-Acetoxyethyl- und 3-Acetoxypropyl-Ester der 2,4-Dichlor-5-fluorzimtsäure.
Bevorzugt sind der Methyl-, Ethyl-, n-Propyl, i-Propyl-, n-Butyl-, i-Butyl-, t-Butyl-, Hexyl-, Octyl- und 2-Ethylhexyl-Ester der 2,4-Dichlor-5-fluorzimtsäure.

Daneben sind auch Verbindungen der drei vorstehend genannten Gruppen (1. X = Cl, Y = Z = F; 2. X = Y = Z = F; 3. X = Y = Cl; Z = F) von Interesse, in denen R¹ für eine gegebenenfalls durch eine Alkoxy- oder Acyloxygruppe substituierten, geradkettigen oder verzweigten Alkylrest mit 1 bis 18, insbesondere 1 bis 12 Kohlenstoffatomen oder für eine -(CH₂)ₙ-OR³ Gruppe, worin n = 2 bis 6 ist und R³ einen Alkylrest mit 1 bis 4 Kohlenstoffatomen darstellt, steht. R² steht insbesondere für Wasserstoff oder eine Methylgruppe, bevorzugt ein Wasserstoffatom.
Beispiele hierfür sind die entsprechenden 2-Hydroxyethyl-, 2-Methoxyethyl-, 2-Ethoxyethyl-, 2-Hydroxypropyl-, 3-Hydroxypropyl-, 3-Methoxypropyl-, 3-Ethoxypropyl-, 4-Methoxybutyl-, 4-Ethoxybutyl-Ester.
Bevorzugt sind die 2-Hydroxyethyl-, 2-Methoxyethyl- und 2-Ethoxyethyl-Ester.

Die halogenierten Zimtsäuren und deren Ester lassen sich herstellen, indem man eine geeignete olefinische Verbindung, insbesondere eine gegebenenfalls substituierte Acrylsäure oder einen gegebenenfalls substituierten Acrylsäureester, in Gegenwart eines Palladium enthaltenden Katalysators mit einem entsprechend substituierten Arylhalogenid oder einer Aryldiazoniumverbindung umsetzt.

Die Aryldiazoniumsalze ermöglichen eine relativ einfache Herstellung der Zimtsäuren und deren Estern. Sie sind Gegenstand der vorliegenden Erfindung und lassen sich durch die Formel 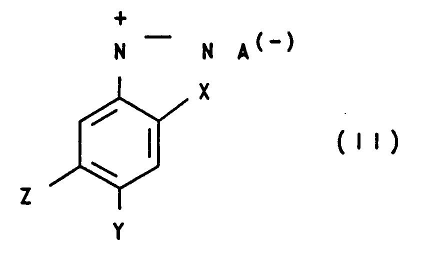 worin zwei der Reste X, Y, und Z gleich sind und für Fluor oder Chlor stehen und der verbleibende Rest Chlor oder Fluor ist oder alle Reste X, Y, Z voneinander verschieden sind und X, Y und Z ein Fluor-, Chlor-, Brom- oder Jodatom und A⁽⁻⁾ ein Anion einer Saure mit einem pKₐ-Wert < 7 bedeuten, beschreiben. Verwendbare Sauren mit einem pKₐ-Wert < 7 sind bspw. HBF₄, HPF₆, H₂SO₄, HCl, HNO₃, CH₃COOH, H₃PO₄, HClO₄, CF₃COOH, CH₃CH₂COOH, Oxalsäure, ClCH₂COOH, PhSO₃H, H₂SnCl₆, insbesondere HBF₄, HPF₆, HCl, H₂SO₄, CH₃COOH, bevorzugt HBF₄, CH₃COOH oder H₂SO₄.

Von besonderer Bedeutung sind Aryldiazoniumverbindungen der vorstehend genannten Formel (II), worin X für ein Chloratom und Y und Z jeweils für ein Fluoratom stehen. Beispiele hierfür sind
2-Chlor-4,5-difluorphenyldiazoniumtetrafluoroborat,
2-Chlor-4,5-difluorphenyldiazoniumhydrogensulfat,
2-Chlor-4,5-difluorphenyldiazoniumsulfat,
2-Chlor-4,5-difluorphenyldiazoniumchlorid,
2-Chlor-4,5-difluorphenyldiazoniumacetat,
Bis-(2-Chlor-4,5-difluorphenyldiazonium)-zinntetrachlorid.

Gleichfalls von besonderer Bedeutung sind Aryldiazoniumverbindungen der vorstehend genannten Formel (II), worin X und Y jeweils für ein Chloratom und Z für ein Fluoratom stehen. Beispiele hierfür sind
2,4-Dichlor-5-fluorphenyldiazoniumtetrafluoroborat,
2,4-Dichlor-5-fluorphenyldiazoniumhydrogensulfat,
2,4-Dichlor-5-fluorphenyldiazoniumsulfat,
2,4-Dichlor-5-fluorphenyldiazoniumchlorid,
2,4-Dichlor-5-fluorphenyldiazoniumacetat,
Bis-(2,4-Dichlor-5-fluorphenyldiazonium)-zinntetrachlorid.

Die Aryldiazoniumverbindungen lassen sich nach gängigen Methoden herstellen (Houben Weyl, Methoden der organischen Chemie, Band X/3, Seiten 3 bis 214, insbesondere 12 bis 113), indem man ein entsprechend durch Halogenatome substituiertes aromatisches Amin mit salpetriger Säure, oder einer salpetrigen Säure erzeugenden Substanz, beispielsweise Alkalinitrit oder einer anderen diazotierenden Substanz, beispielsweise Alkylnitrit, umsetzt. Diese Umsetzung bereitet keine Schwierigkeiten und führt in der Regel zu hohen Ausbeuten. In vielen Fällen empfiehlt es sich, das Aryldiazoniumsalz zu isolieren und in reiner Form, beispielsweise als kristallines Produkt, weiterzuverarbeiten. Es ist auch möglich, das in Lösung hergestellte Diazoniumsalz mittels homogen vorliegender Palladium-Katalysatoren direkt in den jeweiligen Zimtsäuren und Zimtsäureestern umzusetzen.
Arbeitet man mit einem Alkalinitrit, so gestaltet sich die Herstellung der Aryldiazoniumverbindung besonders günstig, wenn man zugleich eine Säure H⁺A⁻, worin A⁻ beispielsweise für BF₄⁻, PF₆⁻, HSO₃⁻, Cl⁻, H₂PO₄⁻, ClO₄⁻ und CH₃COO⁻ steht, verwendet und auf diese Weise bereits den Rest A⁻ in die Aryldiazoniumverbindung einführt.

Die Herstellung von Zimtsäureestern mittels einer durch Palladium katalysierten Umsetzung eines Aryldiazoniumsalzes mit einem Acrylsäurederivat stellt ein erst in jüngerer Zeit näher untersuchtes Verfahren dar.
K. Kikukawa et al., Chem. Lett., 1977, 159; Bull. Chem. Soc., 1979, 52, 2609 und Tetrahedron, 1981, 37, 31 beschreiben die Vinylierung von Aryldiazoniumsalzen in Gegenwart löslicher Pd(O)-Komplexen und einer Base. Die Umsetzungen liefern lediglich bei Einsatz von teurem Bisbenzylidenpalladium (O) in Gegenwart überstöchiometrischer Mengen Base gute Ausbeuten. Zudem werden relativ hohe Mengen (2 mol%) Palladiumkomplex, der nach Beendigung der Reaktion verworfen werden muß, eingesetzt. Die EP 0 508 264 beschreibt ein Verfahren zur Herstellung substituierter Olefine aus Aryldiazoniumsalzen und Olefinen in Gegenwart eines Palladiumkatalysators. Die Synthese von Zimtsäureestern (Beispiele 20 bis 22) erfolgt mittels im Reaktionsgemisch gelöstem Pd(OAc)₂. Die Herstellung halogenierter Zimtsäuren und Zimtsäureester wird im vorstehend genannten Stand der Technik nicht erwähnt. Die Verfahren des Standes der Technik weisen allerdings einen erheblichen Nachteil auf. Der für die Umsetzung benötigte Palladium-Katalysator wird in homogener Form, das heißt in gelöstem Zustand eingesetzt. Dadurch wird eine Abtrennung des Katalysators nach der Umsetzung problematisch. Dies wird zusätzlich noch durch dem Umstand, daß der Palladiumkatalysator in sehr geringen Mengen, nämlich im Bereich von 0,1 bis 5 Mol-%, eingesetzt wird, in beträchtlichem Maße erschwert. Eine Rückgewinnung des wertvollen Palladiumkatalystors und seine Wiederverwertung sehen die Verfahren des Standes der Technik nicht vor, obgleich dies ein für die Durchführung eines technischen Verfahrens wünschenswerter Vorteil sein dürfte.

Die Verwendung von Palladium-Träger-Katalysatoren wird zwar in der EP-A 0 508 264 A1 Seite 4, Zeilen 2 bis 3 erwähnt. Sie wird allerdings lediglich in einem Fall, nämlich bei der Umsetzung von Anilin-2-sulfonsäure mit Ethylen zu Styrol-2-sulfonsäure (Beispiel 6), belegt. Wie ein Vergleich mit dem unter Einsatz von Pd(OAc)₂ durchgeführten Beispiel 4 zeigt, nimmt jedoch die Ausbeute bei Verwendung eines Palladium enthaltenden Trägerkatalysators (10 % Pd auf Kohle) erheblich ab (Beispiel 4: 87 % Ausbeute; Beispiel 7: 74 % Ausbeute). In beiden Beispielen wird die Umsetzung unter Zusatz einer Base, die bezogen auf Anilin-2-sulfonsäure im Überschuß eingesetzt wird, durchgeführt. Wie der experimentelle Befund beweist (siehe Vergleichsversuch im experimentellen Teil), läßt sich die in Beispielen der EP-A 0 508 264 praktizierte Arbeitsweise, die Aryldiazoniumsalze in situ herzustellen und anschließend weiterzuverarbeiten, nicht generell auf eine Herstellung eines Zimtsäureesters mittels eines Palladium enthaltenden Trägerkatalysators übertragen. Der Vergleichsversuch belegt, daß der gewünschte Zimtsäureester nicht einmal in kleinen Mengen gebildet wird. Dieser experimentelle Befund läßt nicht erwarten, daß sich Palladium enthaltende Trägerkatalysatoren für die Herstellung der erfindungsgemäßen Zimtsäuren und Zimtsäureester eignen.

Die vorliegende Erfindung betrifft ferner ein Verfahren zur Herstellung der erfindungsgemäßen Verbindungen. Es ist dadurch gekennzeichnet, daß man ein Aryldiazoniumsalz der Formel 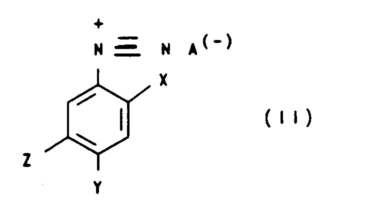 worin die Reste X, Y und Z gleich sind und für ein Fluor-, Brom- oder Jodatom stehen oder, falls zwei der Reste X, Y oder Z gleich oder alle Reste X, Y, Z voneinander verschieden sind, X, Y und Z ein Fluor-, Chlor-, Brom- oder Jodatom und A⁽⁻⁾ ein Anion einer Säure mit einem pKₐ-Wert < 7 bedeuten, mit einer Verbindung der Formel 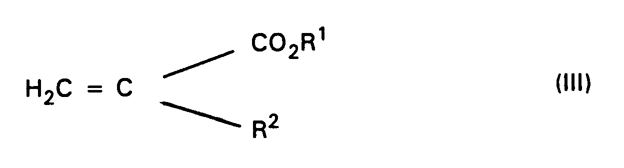 worin R¹ und R² gleich oder verschieden sind und für Wasserstoff oder einen gegebenenfalls Sauerstoff, Stickstoff oder Halogen enthaltenden Alkylrest mit 1 bis 18 Kohlenstoffatomen stehen, in Gegenwart eines Palladium enthaltenden Katalysators, gegebenenfalls unter Zusatz einer Base, umsetzt.

Für das Verfahren sind Aryldiazoniumverbindungen der Formel (II), worin X für ein Chloratom und Y und Z für ein Fluoratom, oder X, Y und Z jeweils für ein Fluoratom oder X und Y jeweils für ein Chloratom und Z für ein Fluoratom und A⁻ für NO₃⁻, BF₄⁻, PF₆⁻, HSO₄⁻, Cl⁻, CH₃COO⁻, insbesondere BF₄⁻, PF₆⁻, HSO₄⁻, Cl⁻, CH₃COO⁻. bevorzugt BF₄⁻ oder HSO₄⁻ stehen, gut geeignet.

Das Verfahren läßt sich sowohl mit Palladium enthaltenden Katalysatoren in homogener Form, die dementsprechend in gelöstem Zustand vorliegen, als auch mit Palladium enthaltenden Katalysatoren in heterogener Form, insbesondere Palladium enthaltenden Trägerkatalystoren, durchführen.
Geeignete homogene Palladiumverbindungen sind beispielsweise Palladiumacetat, Palladiumdichlorid, Palladiumdibromid, Palladiumdinitrat, Natriumtetrachloropalladat und Palladiumsulfat. Sie können allein oder in Form beliebiger Gemische verwendet werden. In bestimmten Fällen kann es von Vorteil sein, bei der Durchführung des erfindungsgemäßen Verfahrens Verbindungen zuzusetzen, die mit Palladium bzw. Palladiumsalzen Komplexe bilden. Geeignete Komplexbildner sind Nitrile wie Benzonitril oder Acetonitril und Phosphite wie Triethylphosphit. Bevorzugt sind Phosphane wie Triarylphosphane und Trialkylphosphane. Auch kann es von Vorteil sein, chelatisierende Phosphane oder Bisphosphane oder Gemische von Bis- und Monophosphanen einzusetzen. Die Phosphane können in organischen Lösungsmitteln und Wasser 2löslich sein, wobei diese im letzten Fall mit ionischen Substituenten versehen sein müssen.
Die Palladiumphosphankomplexe werden bevorzugt in situ erzeugt, es können aber auch vorgebildete Komplexe wie Palladiumtetrakistriphenylphosphan eingesetzt werden.

Ein besonderer Vorteil besteht in der Verwendung Palladium enthaltender Trägerkatalysatoren, die sich nach Abschluß der Umsetzung sehr einfach, beispielsweise durch Filtration oder Dekantieren, abtrennen lassen. Für ein technisches Verfahren bietet es sich an, den Palladium enthaltenden Trägerkatalysator in einem Festbett anzuordnen und das umzusetzende Einsatzgemisch darüberzuleiten. Eine separate Anwendung des als Festbett verwendeten Palladium enthaltenden Katalysators ist nicht mehr erforderlich.

Heterogene Palladiumkatalysatoren sind bspw. metallisches Palladium, Palladium schwarz oder Palladium auf einem Trägermaterial fixiert. Als Trägermaterialien können beliebige inerte Feststoffe eingesetzt werden. Beispielhaft seien hier Aktivkohle, Aluminiumoxide, Siliciumoxide, Magnesiumoxid, Alumosilikate, Kaliumcarbonat, Bariumsulfat und Calciumcarbonat genannt. Besonders geeignete Trägermaterialien sind Aktivkohle, Aluminiumoxide, Siliciumdioxide und Alumosilikate.

Üblicherweise führt man die Umsetzung in Gegenwart eines Lösungsmittels durch. Als Lösungsmittel lassen sich sowohl organische, bevorzugt dipolar aprotische Lösungsmittel als auch protische Lösungsmittel verwenden.

Wird die Reaktion in einem dipolar aprotischen Lösungsmittel durchgeführt so sind folgende Lösungsmittel geeignet: Ether, bevorzugt cyclische Ether wie Tetrahydrofuran oder Dioxan und acyclische Ether wie Methyl-tert.-butylether, Glymes wie Di-, Tri- und Tetraglyme, N, N-Dialkylamide, besonders Dimethylacetamid, Dimethylformamid und N-Methylpyrrolidon. Als protisches Lösungsmittel eignen sich Alkohole, bevorzugt Methanol, Ethanol, Isopropanol, Ethylenglykol, 2-Ethylhexanol und Wasser.

Es können auch Gemische verschiedener Lösungsmittel eingesetzt werden, auch solche, die Mehrphasensysteme bilden.

Üblicherweise führt man das Verfahren bei einer Temperatur von -10 bis 120 °C durch. Man kann in einer Reihe von Fällen bei Temperaturen von 0 bis 100, insbesondere 20 bis 80 °C arbeiten.

Die vorliegende Erfindung betrifft ferner ein weiteres Verfahren zur Herstellung der vorstehend beschriebenen Verbindungen. Es ist dadurch gekennzeichnet, daß man ein Arylhalogenid der Formel 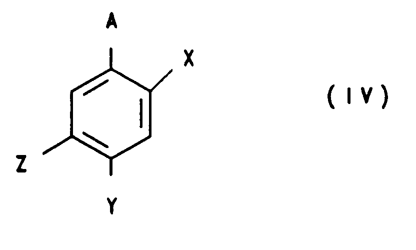 worin A ein Bromatom, X und/oder Y ein Fluoratom oder Chloratom und Z ein Fluoratom ist oder A ein Jodatom X und/oder Y ein Fluoratom, Chloratom oder Bromatom und Z ein Fluoratom ist oder A ein Chloratom, Bromatom oder Jodatom und X, Y und Z jeweils ein Fluoratom ist, mit einer Verbindung 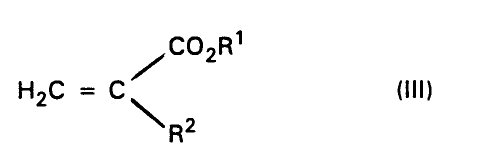 worin R¹ und R² gleich oder verschieden sind und für Wasserstoff oder einen gegebenenfalls Sauerstoff, Stickstoff oder Halogen enthaltenden Alkylrest mit 1 bis 18 Kohlenstoffatomen stehen in Gegenwart eines Palladium enthaltenden Katalysators, gegebenenfalls unter Zusatz einer Base, umsetzt. Die Umsetzung läßt sich sowohl ohne Zusatz eines Lösungsmittels als auch in Gegenwart eines Lösungsmittels durchführen. Üblicherweise verwendet man ein Lösungsmittel.

Geeignete inerte organische Lösungsmittel sind je nach Reaktionskomponenten z.B. gegebenenfalls chlorierte aliphatische, cycloaliphatische oder aromatische Kohlenwasserstoffe, wie n-Pentan, n-Heptan, n-Octan, Cyclopentan, Cyclohexan, Benzol, Toluol, Xylole und Chlorbenzol; aromatische, aliphatische und cyclische Ether, wie Anisol, Diethylether, Diisopropylether, Tetrahydrofuran und Dioxan; N-substituierte Morpholine, wie N-Methyl- und N-Formylmorpholin; Nitrile, besonders Benzonitril und Alkylnitrile mit 2 bis 5 C-Atomen, wie Acetonitril, Propionitril, Butyronitril-, 3-Methoxypropionitril und 3-Ethoxypropionitril; Dialkylsulfoxide, wie Dimethyl- und Diethylsulfoxid; N,N-Dialkylamide von aliphatischen Monocarbonsäuren mit 1 bis 3 C-Atomen im Säureteil, wie N,N-Dimethylformamid und N,N-Dimethylacetamid; Alkohole mit bis zu 8 C-Atomen, wie Ethanol, n-Propanol und tert.-Butanol; aliphatische und cyclische Ketone, wie Aceton, Diethylketon, Methylisopropylketon, Cyclopentanon, Cyclohexanon, 1,3-Dimethyl-2-imidazolidinon und 1,3-Dimethyl-3,4,5,6-tetrahydro-2-(1H)-pyrimidinon; Tetramethylharnstoff; Ester, wie Ester der Kohlensäure, z.B. Diethylcarbonat; Nitromethan; Alkyl- oder Alkoxyalkylester von aliphatischen Monocarbonsäuren mit insgesamt 2 bis 8 C-Atomen, wie Essigsäuremethyl-, -ethyl-, -n-butyl- und -isobutylester, Buttersäureethyl-und n-butylester sowie 1-Acetoxy-2-ethoxyethan. Bevorzugte Lösungsmittel sind N,N-Dialkylamide, besonders Dimethylacetamid, Dimethylformamid, N-Methylpyrrolidon und Ethylengykol-, Di-, Tri- und Tetraethylenglykoldimethylether. Bevorzugt sind als Lösungsmittel dipolar aprotische Lösungsmittel.

Es können auch Gemische der Lösungsmittel eingesetzt werden, auch solche, die Mehrphasensysteme bilden.

Es ist von Vorteil, 2,4,5-Trihalogenarylhalogenide mit Acrylsäure oder Acrylsäureestern in Gegenwart von Basen reagieren zu lassen.

Geeignete Basen sind offenkettige oder cyclische sekundäre oder tertiäre Amine, wie Diethylamin, Triethylamin, Pyrrolidin, Piperidin, Piperazin, Diazabicyclooctan (DABCO), Diazabicyclononen (DBN), Diazabicycloundecan (DBU), Arylamine, Aryldiamine, Alkali- und Erdalkalisalze von aliphatischen und aromatischen Carbonsäuren, wie Natrium-, Kalium- oder Calciumacetat, Natrium- oder Kaliumpropionat, Natrium- oder Kaliumlaurat, Natrium- oder Kaliumbenzoat, Alkali- sowie Erdalkalicarbonate, wie Kaliumcarbonat, Natriumcarbonat, Lithiumcarbonat, Calciumcarbonat, Alkali- bzw. Erdalkalihydrogencarbonate, wie Natriumhydrogencarbonat, Calciumhydrogencarbonat oder auch Alkali- oder Erdalkalihydroxide, wie Lithiumhydroxid, Kaliumhydroxid, Natriumhydroxid, Calciumhydroxid, Bariumhydroxid. Die genannten Basen können alleine oder in beliebigen Mischungen untereinander eingesetzt werden.

Zweckmäßigerweise führt man die Reaktion bei erhöhter Temperatur durch. Üblicherweise arbeitet man bei Temperaturen von 50 bis 250 °C. In den meisten Fällen hat sich eine Temperatur von 60 bis 200, insbesondere 80 bis 180 °C für die Durchführung der Umsetzung als ausreichend erwiesen.

Als Katalysatoren eignen sich Palladium enthaltende Katalysatoren sowohl in homogener Form als auch in heterogener Form.
Geeignete homogene Palladiumverbindungen sind Palladiumacetat, Palladiumdichlorid, Palladiumdibromid, Palladiumdinitrat und Palladiumsulfat. Sie können allein oder in beliebigen Gemischen verwendet werden. Bei der Umsetzung der 2,4,5-Trihalogenarylhalogenide kann es von Vorteil sein, Verbindungen zuzusetzen, die mit Palladium oder Palladiumsalzen Komplexe bilden. Geeignete Komplexbildner sind Nitrile wie Benzonitril oder Acetonitril und Phosphite wie Triethylphosphit. Bevorzugt sind Phosphane.

Als einzähnige Monophosphane kommen insbesondere Triarylphosphane, Dialkylarylphosphane, Diarylphosphane, Diarylalkylphosphane und Trialkylphosphane in Frage, wobei die Alkylgruppen 1 bis 12 C-Atome enthalten und die Arylgruppen Phenyl- oder Naphthylgruppen sind, die jeweils mit C₁₋₄-Alkyl, C₁₋₃-Alkoxy oder SO₃Na substituiert sein können.

Als Beispiele seien genannt:
Triphenylphosphan, Tricyclohexylphosphan, Triisopropylphosphan, Tri-n-butylphosphan, Tri(methoxyphenyl)phosphan, Diisopropylphenylphosphan, Diphenylisopropylphosphan, Triisobutylphosphan, Methyldiphenylphosphan, Tri-o- und Tri-p-tolylphosphan, Triethylphosphan, tert.-Butyldiphenylphosphan, und Tri-(sulfonatophenyl)phosphan.

Besonders bevorzugt sind Triphenylphosphan, Tricyclohexylphosphan und Tri-o-tolyl-phosphan.

Auch kann es von Vorteil sein, chelatisierende Phosphane oder Bisphosphane oder Gemische von Bis- und Monophosphanen einzusetzen. Die Phosphane können in organischen Lösungsmitteln und Wasser löslich sein, wobei diese im letzten Fall mit ionischen Substituenten versehen sein müssen.

Die Palladiumphosphankomplexe werden bevorzugt in situ erzeugt, es können aber auch vorgebildete Komplexe, wie Palladiumtetrakistriphenylphosphan, eingesetzt werden.
Heterogene Palladiumkatalysatoren sind metallisches Palladium, Palladiumschwarz oder Palladium auf einem Trägermaterial fixiert. Als Trägermaterialien können beliebige inerte Feststoffe eingesetzt werden. Beispielhaft seien hier Aktivkohle, Aluminiumoxide, Siliciumoxide, Magnesiumoxid, Alumosilikate, Kaliumcarbonat, Bariumsulfat und Calciumcarbonat genannt. Besonders geeignete Trägermaterialien sind Aktivkohle, Aluminiumoxide, Siliciumdioxide.

Die Menge des eingesetzten Palladiums beträgt zweckmäßigerweise 0.001 bis 10 Mol%, bevorzugt 0.01 bis 5 Mol%, bezogen auf das Arylhalogenid.

Der Gehalt des heterogenen Katalysators an Palladium beträgt 1 bis 20 Gew.-%, bevorzugt 2 bis 10 Gew.-%, bezogen auf das Trägermaterial.

Die Umsetzung läßt sich generell bei Unterdruck, Atmosphärendruck oder Überdruck durchführen.

Die nachfolgenden Beispiele belegen die Erfindung, ohne sie zu beschränken.

### Experimenteller Teil

### Vergleichsversuch (analog zu EP 0 508 264 Beispiel 22)

Man vermischt unter Rühren 12,3 g 4-Anisidin (4-Methoxyanilin) mit 10 ml konzentrierter Schwefelsäure und 60 ml 2-Ethylhexanol und kühlt die Mischung auf etwa 10 °C ab. Anschließend gibt man langsam 11,7 g Amylnitrit zu und rüht nach beendeter Zugabe noch 40 Minuten. Das Reaktionsgemisch wird nachfolgend mit 20,2 g Acrylsäure-2-ethylhexylester und 100 mg Pd in (5 Gew.-% Pd auf Aktivkohle) versetzt und binnen 1 Stunde auf 65 °C erwärmt und 12 Stunden bei dieser Temperatur gerührt. Man verdünnt mit 100 ml Wasser und extrahiert mit 200 ml Dichlormethan. Wie Untersuchungen mittels Dünnschichtchromatograhie und Gaschromatographie zeigen, hat sich p-Methoxyzimtsäure-2-ethylhexylester nicht einmal in kleinsten Mengen gebildet. Der eingesetzte Acrylsäure-2-ethylhexylester liegt im wesentlichen in unveränderter Form vor.

### Beispiel 1

8.78 g (31.5 mmol) 2,4-Dichlor-5-fluorphenyldiazoniumtetrafluoroborat, 11.61 g (63.0 mmol) Arylsäure-2-ethylhexylester werden in 40 ml Dimethylsulfoxid suspendiert und mit 0.75 g (0.32 mmol) Palladium (5 Gew.-% Pd auf Aktivkohle) bei 0 °C versetzt. Die Reaktionsmischung wird binnen einer Stunde auf 60 °C erwärmt und 12 Stunden bei dieser Temperatur gerührt. Nach Abkühlung auf Raumtemperatur wird der Katalysator abfiltriert und mit Ethanol gewaschen.
Man verdünnt mit 100 ml Dichlormethan und wäscht dreimal mit 60 ml Wasser.
Die organische Phase wird im Vakuum eingeengt. Das anfallende Rohprodukt wird säulenchromatographiert.
Ausbeute: 84 % 2,4-Dichlor-5-fluorzimtsäure-2-ethylhexylester.
R_{f}: 0.73 (Essigsäureethylester/Petrolether 1:8).
Siedepunkt extrapoliert aus GC-Daten: 363 °C.
¹H-NMR (300 MHz, CDCl₃): 0.90, 0.92 (2t, J = 7.5 Hz, 6H, CH₃), 1.26 - 1.46 (m, 8H, CH₂), 1.57 - 1.70 (m, 1H, CH), 4.15 (dd, J = 2, 7.5 Hz, 2H, CH₂O), 6.42 (d, J = 16 Hz, 1H, CHCHCO₂), 7.42 (d, J = 9 Hz, 1H, CH), 7.47 (d, J = 7 Hz, 1H, CH), 7.95 (dd, J = 2, 16 Hz, 1H, CHCHCO₂).
¹⁹F NMR (94 MHz): 116.9 (CF)
Massenspektrum (EI, 70 eV): 349 (38), 347 (63) (M+H), 219 (58), 217 (100) (C₉H₄OCl₂F), 112 (78) (C₈H₁₆).

### Beispiel 2

Man verfährt wie in Beispiel 1, als Katalysatorsystem dient 1 mol% Palladium bezogen auf Diazoniumsalz (5 Gew.-% auf Aluminiumoxid).
Ausbeute: 80 % 2,4-Dichlor-5-fluorzimtsäure-2-ethylhexylester.

### Beispiel 3

8.78 g (31.5 mmol) 2,4-Dichlor-5-fluorphenyldiazoniumtetrafluoroborat, 8.07 g (63.0 mmol) Acrylsäurebutylester werden in 40 ml Dimethylsulfoxid suspendiert und mit 0.75 g (0.32 mmol) Palladium (5 Gew.-% auf Aktivkohle) bei 0 °C versetzt. Die Reaktionsmischung wird binnen einer Stunde auf 60 °C erwärmt und 12 Stunden bei dieser Temperatur gerührt.
Nach Abkühlung auf Raumtemperatur wird der Katalysator abfiltriert und mit Ethanol gewaschen.
Man verdünnt mit 100 ml Dichlormethan und wäscht dreimal mit 60 ml Wasser.
Die organische Phase wird im Vakuum eingeengt. Das anfallende Rohprodukt wird säulenchromatographiert.
Ausbeute: 73 % 2,4-Dichlor-5-fluorzimtsäurebutylester.
R_{f}: 0.65 (Essigsäureethylester/Petrolether 1:8).
Siedepunkt: 155 - 160 °C bei 0,8 mbar.
¹H NMR (100 MHz, CDCl₃): 0.94 (t, J = 7 Hz, 3H, CH₃), 1.22 - 1.82 (m, 4H, CH₂), 4.24 (t, J = 7 Hz, 2H, CH₂O), 6.40 (d, J = 16.5 Hz, 1H, CHCO₂), 7.43 (d, J = 16.5 Hz, 1H, CH), 7.44 (s, 1H, CH), 7.93 (dd, J = 2, 16.5 Hz, 1H, CHCHCO₂).
¹⁹F NMR (94 MHz): 117.0 (C-F)

### Beispiel 4

8.78 g (31.5 mmol) 2,4-Dichlor-5-fluorphenyldiazoniumtetrafluoroborat, 8.07 g (63.0 mmol) Acrylsäureethylester werden in 40 ml Ethanol suspendiert und mit 0.75 g (0.32 mmol) Palladium auf Aktivkohle (5 %ig) bei 0 °C versetzt. Die Reaktionsmischung wird binnen einer Stunde auf 60 °C erwärmt und 12 Stunden bei dieser Temperatur gerührt.
Nach Abkühlung auf Raumtemperatur wird der Katalysator abfiltriert und mit Ethanol gewaschen.

Das Lösungsmittel wird im Vakuum evaporiert. Das anfallende Rohprodukt enthält 87 % 2,4-Dichlor-5-fluorzimtsäureethylester.
Ausbeute: 93 % 2,4-Dichlor-5-fluorzimtsäureethylester.
R_{f}: 0.59 (Essigsäureethylester/Petrolether 1:8).
Siedepunkt: 147 - 152 °C bei 0.5 Torr.
¹H NMR (100 MHz, CDCl₃): 1.32 (t, J = 7.5 Hz, 3H, CH₃), 4.28 (q, J = 7.5 Hz, 2H, CH₂), 6.37 (d, J = 16 Hz, 1H, CHCO₂), 7.40 (d, J = 16.5 Hz, 1H, CH), 7.43 (s, 1H, CH), 7.91 (dd, J = 2, 16 Hz, 1H, CHCHCO₂).

### Beispiel 5

50.0 g 1-Brom-2,4-dichlor-5-fluorbenzol werden unter Schutzgas mit 65.4 ml Acrylsäurebutylester, 450 mg Palladiumacetat, 1.075 g Triphenylphosphan, 0.815 g Bisphenylphosphanethan und 20.15 g Natriumacetat versetzt und in 100 ml Dimethylacetamid gelöst. Die Reaktionsmischung wird 12 Stunden bei 140 - 145 °C gekocht. Anschließend wird mit 200 ml Dichlormethan verdünnt und zweimal mit 100 ml Wasser ausgeschüttet. Die organische Phase wird mittels eines Rotationsverdampfers eingeengt.
Ausbeute: 86 % 2,4-Dichlor-5-fluorzimtsäurebutylester.

### Beispiel 6

### Synthese von 2,4-Dichlor-5-fluorphenyldiazoniumtetrafluoroborat

100 g 2,4-Dichlor-5-fluoranilin werden bei Raumtemperatur mit 165 ml Tetrahydrofuran versetzt. Zu dieser Lösung gibt man 333 ml Tetrafluorborsäure (50 %ig) unter Kühlung zu. Die auf 5-10°C abgekühlte Reaktionsmischung wird mit 57.5 g Natriumnitrit in 117 ml Wasser so versetzt, daß die Innentemperatur 7-13°C beträgt. Nach vollendeter Zugabe filtriert man die 0°C kalte Lösung. Der Rückstand wird mit Tetrahydrofuran und eiskaltem Wasser gewaschen. Es verbleiben 121 g Produkt.
Ausbeute: 80 % 2,4-Dichlor-5-fluorphenyldiazoniumtetrafluoroborat
¹H NMR (100 MHz, D₂O): 8.31 (d, J = 6 Hz, 1H, CH), 8.65 (d, 6.5 Hz, 1H, CH)
¹⁹F NMR (d₆-Aceton): 100.8 (t, CF), 151.3 (s, BF₄)

### Beispiel 7

### Alternativsynthese für 2,4-Dichlor-5-fluorphenyldiazoniumtetrafluoroborat

16 g 2,4-Dichlor-5-fluoranilin werden mit 160 ml Tetrafluorborsäure auf 100°C erwärmt. Die orange-braune Lösung wird dann auf 0-5°C abgekühlt und mit 10.5 g Natriumnitrit in 25 ml Wasser tropfenweise versetzt. Anschließend gibt man die Reaktionslösung auf 200 ml Eiswasser. Das Produkt wird abgesaugt und mit Tetrahydrofuran gewaschen.
Ausbeute: 60 % 2,4-Dichlor-5-fluorphenyldiazoniumtetrafluoroborat

### Beispiel 8

### Synthese von 2,4,5-Trifluorzimtsäureethylhexylester

3.0 g (14.2 mmol) 1-Brom-2,4,5-trifluorbenzol werden unter Schutzgas mit 30.0 mmol Acrylsäure-2-ethylhexylester, 140 mg Palladium auf Aktivkohle (5 %ig), 0.82 g Natriumcarbonat und 10 mg Ditert.butylphenyl in 10 ml Dimethylacetamid 18 Stunden bei 170°C refluxiert. Man filtriert den Katalysator ab, verdünnt mit Dichlormethan und extrahiert mit Wasser. Das Rohprodukt wird chromatographiert.
Ausbeute: 87 % 2,4,5-Trifluorzimtsäureethylhexylester
R_{f}: = (Essigsäureethylester/Petrolether 1:6)
¹H-NMR (CDCl₃): 0.91, 0.93 (2t, J = 7.5 Hz, 6H, CH₃), 1.27-1.46 (m, 8H, CH₂), 1.59-1.72 (m, 1H, CH), 4.17 (dd, J = 2, 7.5 Hz, 2H, CH₂O), 6.46 (d, J = 16 Hz, 1H, CHCHCO₂), 7.46-7.55 (m, 2H, CH), 7.99 (dd, J = 2, 16 Hz, 1H, CHCHCO₂).
MS (70eV): 315 (100 %) M⁺

### Beispiel 9

### Synthese von 2,4-Dichlor-5-fluorphenyldiazoniumhydrogensulfat

36.0 g (0.20 mol) 2,4-Dichlor-5-fluoranilin werden bei Raumtemperatur in 180 ml Ethanol gelöst und unter Kühlung mit 36.0 g konz. Schwefelsäure versetzt. Zu dieser Mischung addiert man tropfenweise 27.0 (0.23 mol) Amylnitrit. Anschließend wird das ausgefallene Diazoniumsalz abgesaugt und mit wenig Diethylether gewaschen.
Ausbeute: 74 % 2,4-Dichlor-5-fluorphenyldiazoniumhydrogensulfat
¹H NMR (100 MHz, d₆-Aceton): 8.83 (d, J = 7 Hz, 1H, CH), 8.94 (d, J = 7 Hz, 1 CH, CH)

## Patentansprüche

1. Verbindungen der Formel 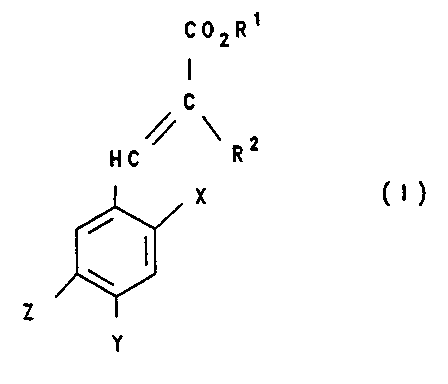 worin R¹ und R² gleich oder verschieden sind und für Wasserstoff oder einen gegebenenfalls Sauerstoff, Stickstoff oder Halogen enthaltenden Alkylrest mit 1 bis 18 Kohlenstoffatomen stehen, die Reste X, Y und Z gleich sind und für ein Fluor-, Brom- oder Jodatom stehen oder, falls zwei der Reste X, Y oder Z gleich oder alle Reste X, Y, Z voneinander verschieden sind, X, Y und Z ein Fluor-, Chlor-, Brom oder Jodatom bedeuten.

2. Verbindungen nach Anspruch 1, dadurch gekennzeichnet, daß R² für Wasserstoff oder eine Methylgruppe steht.

3. Verbindungen nach einem oder mehreren der Ansprüche 1 und 2, dadurch gekennzeichnet, daß R¹ für einen gegebenenfalls durch eine Alkoxygruppe oder Acyloxygruppe substitutierten geradkettigen oder verzweigten Alkylrest mit 1 bis 18, insbesondere 1 bis 12 Kohlenstoffatomen steht.

4. Verbindungen nach einem oder mehreren der Ansprüche 1 bis 3, dadurch gekennzeichnet, daß R¹ für eine -(CH₂)ₙ-OR³ Gruppe steht, worin n = 2 bis 6 ist und R³ einen Alkylrest mit 1 bis 4 Kohlenstoffatomen bedeutet.

5. Verbindungen nach einem oder mehreren der Ansprüche 1 bis 4, dadurch gekennzeichnet, daß X und Y jeweils für ein Bromatom und Z für ein Fluoratom stehen.

6. Verbindungen nach einem oder mehreren der Ansprüche 1 bis 4, dadurch gekennzeichnet, daß X für ein Bromatom, Y und Z jeweils für ein Fluoratom oder Y für ein Chloratom und Z für ein Fluoratom oder X für ein Chloratom, Y für ein Bromatom und Z für ein Fluoratom stehen, oder X und Z für ein Fluoratom und Y für ein Brom- oder Chloratom stehen.

7. Verbindungen nach Anspruch 1, dadurch gekennzeichnet, daß sie der Formel 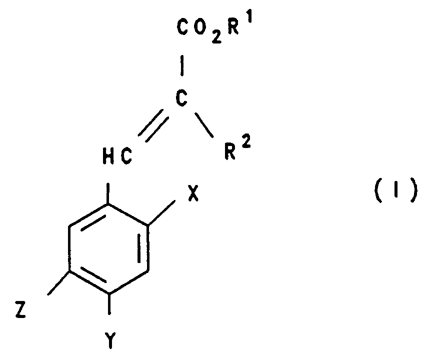 entsprechen, worin R¹ und R² gleich oder verschieden sind und für Wasserstoff oder einen gegebenenfalls Sauerstoff, Stickstoff oder Halogen enthaltenden Alkylrest mit 1 bis 18 Kohlenstoffatomen, X für ein Chloratom und Y und Z jeweils für ein Fluoratom stehen.

8. Verbindungen nach Anspruch 1, dadurch gekennzeichnet, daß sie der Formel 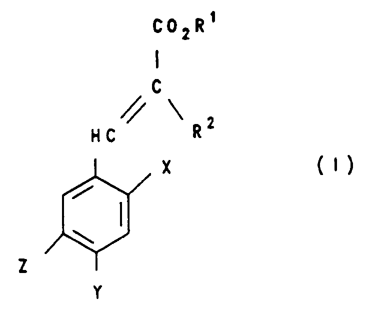 entsprechen, worin R¹ und R² gleich oder verschieden sind und für Wasserstoff oder einen gegebenenfalls Sauerstoff, Stickstoff oder Halogen enthaltenden Alkylrest mit 1 bis 18 Kohlenstoffatomen stehen und X, Y und Z jeweils für ein Fluoratom stehen.

9. Verbindungen nach Anspruch 1, dadurch gekennzeichnet, daß sie der Formel 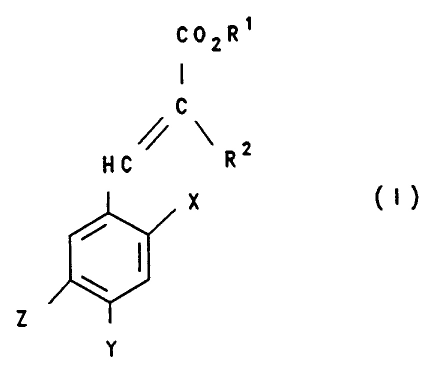 entsprechen, worin R¹ und R² gleich oder verschieden sind und für Wasserstoff oder einen gegebenenfalls Sauerstoff, Stickstoff oder Halogen enthaltenden Alkylrest mit 1 bis 18 Kohlenstoffatomen und X und Y jeweils für ein Chloratom und Z für ein Fluoratom stehen.

10. Verbindungen nach einem der Ansprüche 7 bis 9, dadurch gekennzeichnet, daß R² für Wasserstoff oder eine Methylgruppe steht.

11. Verbindungen nach einem der Ansprüche 7 bis 10, dadurch gekennzeichnet, daß R¹ für eine gegebenenfalls durch eine Alkoxy- oder Acyloxygruppe substituierten, geradkettigen oder verzweigten Alkylrest mit 1 bis 18, insbesondere 1 bis 12 Kohlenstoffatomen steht.

12. Verbindungen nach einem oder mehreren der Ansprüche 7 bis 11, dadurch gekennzeichnet, daß R¹ für eine -(CH₂)ₙ-OR³ Gruppe steht, worin n = 2 bis 6 ist und R³ einen Alkylrest mit 1 bis 4 Kohlenstoffatomen bedeutet.

13. Aryldiazoniumverbindungen der Formel 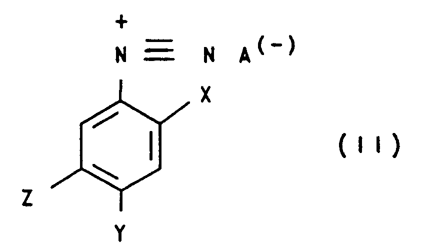 worin zwei der Rest X, Y oder Z gleich sind und für Fluor oder Chlor stehen und der verbleibende Rest Chlor oder Fluor ist oder alle Reste X, Y und Z voneinander verschieden sind und X, Y und Z ein Fluor-, Chlor-, Brom- oder Jodatom und A⁽⁻⁾ ein Anion einer Säure mit einem pKₐ-Wert < 7 bedeuten.

14. Aryldiazoniumverbindungen nach Anspruch 13, dadurch gekennzeichnet, daß sie der Formel 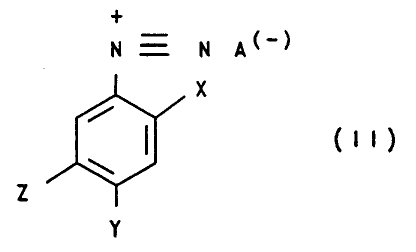 entsprechen, worin X für ein Chloratom und Y und Z jeweils für ein Fluoratom stehen.

15. Aryldiazoniumverbindungen nach Anspruch 13, dadurch gekennzeichnet, daß sie der Formel 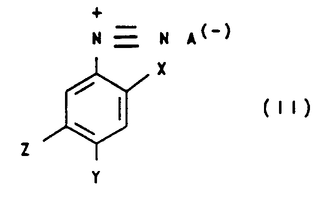 entsprechen, worin X und Y jeweils für ein Chloratom und Z für ein Fluoratom stehen.

16. Verfahren zur Herstellung der Verbindungen nach einem oder mehreren der Ansprüche 1 bis 12, dadurch gekennzeichnet, daß man ein Aryldiazoniumsalz der Formel 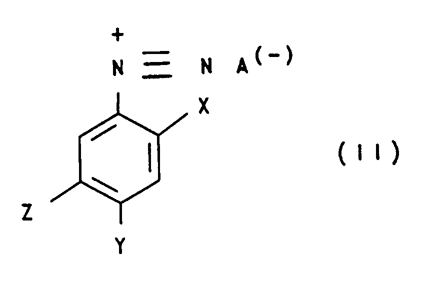 worin die Reste X, Y und Z gleich sind und für ein Fluor-, Brom- oder Jodatom stehen oder, falls zwei der Reste X, Y und Z gleich oder alle Reste X, Y, Z voneinander verschieden sind, X, Y und Z ein Fluor-, Chlor-, Brom- oder Jodatom und A⁽⁻⁾ ein Anion einer Säure mit einem pKₐ-Wert < 7 bedeuten, mit einer Verbindung der Formel 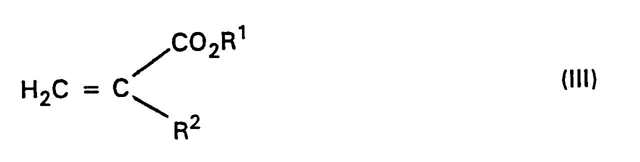 worin R¹ und R² gleich oder verschieden sind und für Wasserstoff oder einen gegebenenfalls Sauerstoff, Stickstoff oder Halogen enthaltenden Alkylrest mit 1 bis 18 Kohlenstoffatomen stehen, in Gegenwart eines Palladium enthaltenden Katalysators, gegebenenfalls unter Zusatz einer Base, umsetzt.

17. Verfahren nach Anspruch 16, dadurch gekennzeichnet, daß man die Umsetzung in Gegenwart eines Lösungsmittels durchführt.

18. Verfahren nach einem oder mehreren der Ansprüche 16 und 17, dadurch gekennzeichnet, daß man als Lösungsmittel ein dipolares aprotisches oder ein protisches Lösungsmittel einsetzt.

19. Verfahren nach einem oder mehreren der Ansprüche 16 bis 18, dadurch gekennzeichnet, daß man die Umsetzung bei Temperaturen von -20 bis 120, insbesondere -10 bis 100, bevorzugt 0 bis 80 °C durchführt.

20. Verfahren nach einem oder mehreren der Ansprüche 16 bis 19, dadurch gekennzeichnet, daß man die Umsetzung in Gegenwart eines Palladium enthaltenden Trägerkatalysators durchführt.

21. Verfahren zur Herstellung der Verbindungen nach einem oder mehreren der Ansprüche 1 bis 12, dadurch gekennzeichnet, daß man ein Arylhalogenid der Formel 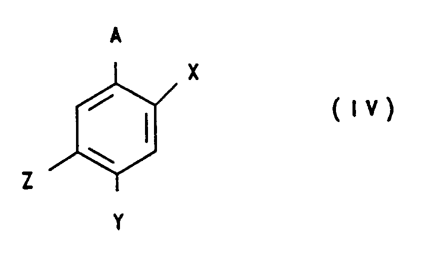 worin A ein Bromatom, X und/oder Y ein Fluoratom oder Chloratom und Z ein Fluoratom ist oder A ein Jodatom X und/oder Y ein Fluoratom, Chloratom oder Bromatom und Z ein Fluoratom ist oder A ein Chloratom, Bromatom oder Jodatom und X, Y und Z jeweils ein Fluoratom ist, mit einer Verbindung der Formel 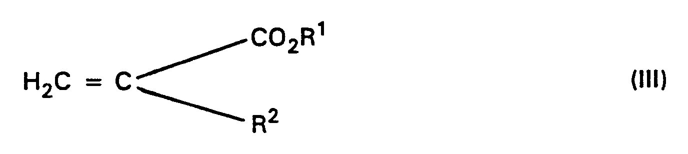 worin R¹ und R² gleich oder verschieden sind und für Wasserstoff oder einen gegebenenfalls Sauerstoff, Stickstoff oder Halogen enthaltenden Alkylrest mit 1 bis 18 Kohlenstoffatomen stehen, in Gegenwart eines Palladium enthaltenden Katalysators, gegebenenfalls unter Zusatz einer Base, umsetzt.

22. Verfahren nach Anspruch 21, dadurch gekennzeichnet, daß man die Umsetzung in Anwesenheit eines Lösungsmittels durchführt.

23. Verfahren nach einem oder mehreren der Ansprüche 21 und 22, dadurch gekennzeichnet, daß man als Lösungsmittel ein dipolar aprotisches Lösungsmittel einsetzt.

24. Verfahren nach einem oder mehreren der Ansprüche 21 bis 23, dadurch gekennzeichnet, daß man die Umsetzung bei Temperaturen von 50 bis 250, insbesondere 60 bis 200, bevorzugt 80 bis 180 °C durchführt.

## Claims

1. A compound of the formula 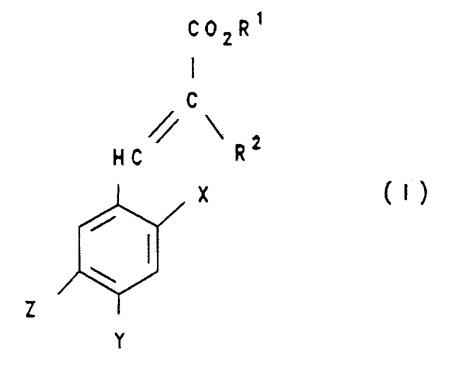 in which R¹ and R² are identical or different and are hydrogen or an alkyl radical having 1 to 18 carbon atoms and optionally containing oxygen, nitrogen or halogen, the radicals X, Y and Z are identical and are a fluorine, bromine or iodine atom or, if two of the radicals X, Y or Z are identical or all of the radicals X, Y, Z are different from each other, X, Y and Z are a fluorine, chlorine, bromine or iodine atom.

2. A compound as claimed in claim 1, wherein R² is hydrogen or a methyl group.

3. A compound as claimed in one or more of claims 1 and 2, wherein R¹ is a straight-chain or branched alkyl radical having 1 to 18, in particular 1 to 12, carbon atoms and optionally substituted by an alkoxy group or acyloxy group.

4. A compound as claimed in one or more of claims 1 to 3, wherein R¹ is a -(CH₂)ₙ-OR³ group, in which n = 2 to 6 and R³ is an alkyl radical having 1 to 4 carbon atoms.

5. A compound as claimed in one or more of claims 1 to 4, wherein X and Y are each a bromine atom and Z is a fluorine atom.

6. A compound as claimed in one or more of claims 1 to 4, wherein X is a bromine atom, Y and Z are each a fluorine atom or Y is a chlorine atom and Z is a fluorine atom or X is a chlorine atom, Y is a bromine atom and Z is a fluorine atom, or X and Z are a fluorine atom and Y is a bromine or chlorine atom.

7. A compound as claimed in claim 1, which corresponds to the formula 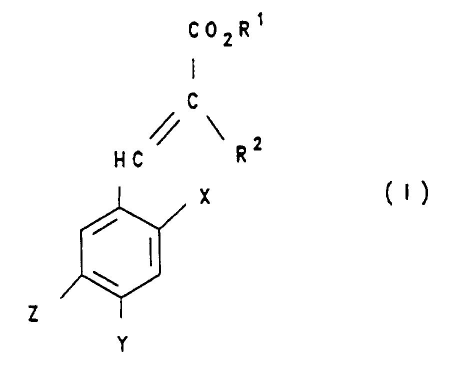 in which R¹ and R² are identical or different and are hydrogen or an alkyl radical having 1 to 18 carbon atoms and optionally containing oxygen, nitrogen or halogen, X is a chlorine atom and Y and Z are each a fluorine atom.

8. A compound as claimed in claim 1, which corresponds to the formula 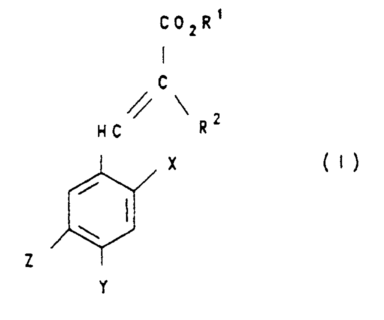 in which R¹ and R² are identical or different and are hydrogen or an alkyl radical having 1 to 18 carbon atoms and optionally containing oxygen, nitrogen or halogen, and X, Y and Z are each a fluorine atom.

9. A compound as claimed in claim 1, which corresponds to the formula 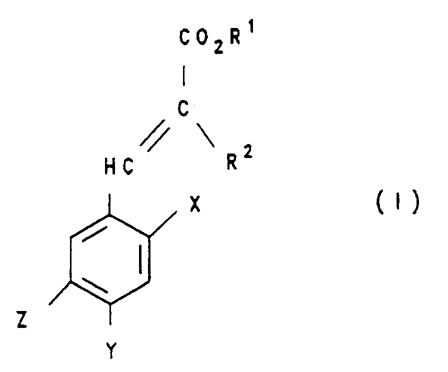 in which R¹ and R² are identical or different and are hydrogen or an alkyl radical having 1 to 18 carbon atoms and optionally containing oxygen, nitrogen or halogen, and X and Y are each a chlorine atom and Z is a fluorine atom.

10. A compound as claimed in one of claims 7 to 9, wherein R² is hydrogen or a methyl group.

11. A compound as claimed in one of claims 7 to 10, wherein R¹ is a straight-chain or branched alkyl radical having 1 to 18, in particular 1 to 12, carbon atoms and optionally substituted by an alkoxy or acyloxy group.

12. A compound as claimed in one or more of claims 7 to 11, wherein R¹ is a -(CH₂)ₙ-OR³ group, in which n = 2 to 6 and R³ is an alkyl radical having 1 to 4 carbon atoms.

13. An aryldiazonium compound of the formula 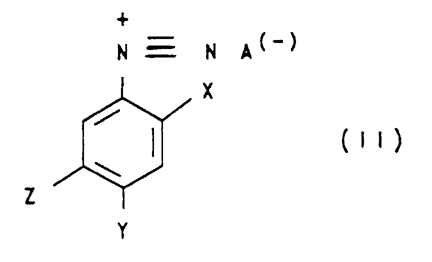 in which two of the radicals X, Y and Z are identical and are fluorine or chlorine and the remaining radical is chlorine or fluorine or all of the radicals X, Y and Z are different from each other and X, Y and Z are a fluorine, chlorine, bromine or iodine atom and A⁽⁻⁾ is an anion of an acid having a pKₐ < 7.

14. An aryldiazonium compound as claimed in claim 13, which corresponds to the formula 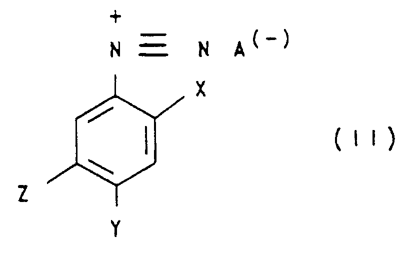 in which X is a chlorine atom and Y and Z are each a fluorine atom.

15. An aryldiazonium compound as claimed in claim 13, which corresponds to the formula 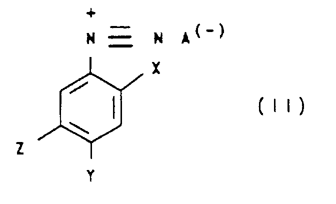 in which X and Y are each a chlorine atom and Z is a fluorine atom.

16. A process for the preparation of the compounds as claimed in one or more of claims 1 to 12, which comprises reacting an aryldiazonium salt of the formula 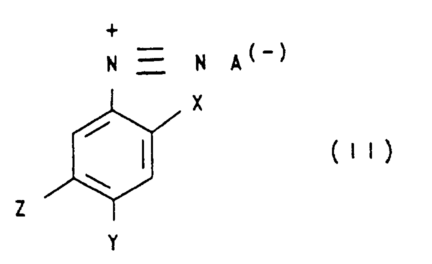 in which the radicals X, Y and Z are identical and are a fluorine, bromine or iodine atom or, if two of the radicals X, Y and Z are identical or all of the radicals X, Y, Z are different from each other, X, Y and Z are a fluorine, chlorine, bromine or iodine atom and A⁽⁻⁾ is an anion of an acid having a pKₐ < 7, with a compound of the formula 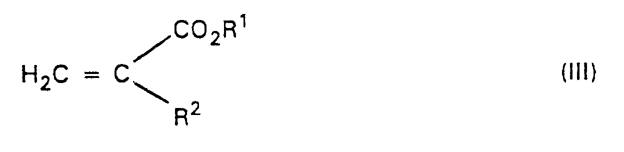 in which R¹ and R² are identical or different and are hydrogen or an alkyl radical having 1 to 18 carbon atoms and optionally containing oxygen, nitrogen or halogen, in the presence of a palladium-containing catalyst, if appropriate with addition of a base.

17. The process as claimed in claim 16, wherein the reaction is carried out in the presence of a solvent.

18. The process as claimed in one or more of claims 16 and 17, wherein the solvent used is a dipolar aprotic or a protic solvent.

19. The process as claimed in one or more of claims 16 to 18, wherein the reaction is carried out at temperatures of -20 to 120, in particular -10 to 100, preferably 0 to 80, °C.

20. The process as claimed in one or more of claims 16 to 19, wherein the reaction is carried out in the presence of a palladium-containing supported catalyst.

21. A process for the preparation of the compounds as claimed in one or more of claims 1 to 12, which comprises reacting an aryl halide of the formula 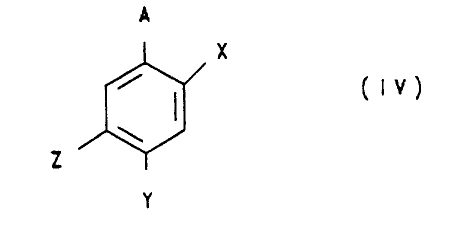 in which A is a bromine atom, X and/or Y is a fluorine atom or chlorine atom and Z is a fluorine atom or A is an iodine atom, X and/or Y is a fluorine atom, chlorine atom or bromine atom and Z is a fluorine atom or A is a chlorine atom, bromine atom or iodine atom and X, Y and Z are each a fluorine atom, with a compound of the formula 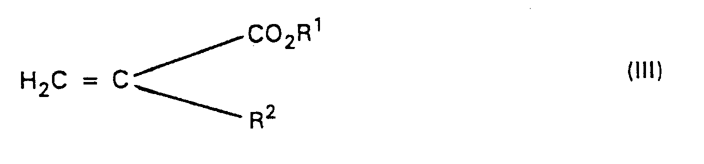 in which R¹ and R² are identical or different and are hydrogen or an alkyl radical having 1 to 18 carbon atoms and optionally containing oxygen, nitrogen or halogen, in the presence of a palladium-containing catalyst, if appropriate with addition of a base.

22. The process as claimed in claim 21, wherein the reaction is carried out in the presence of a solvent.

23. The process as claimed in one or more of claims 21 and 22, wherein the solvent used is a dipolar aprotic solvent.

24. The process as claimed in one or more of claims 21 to 23, wherein the reaction is carried out at temperatures of 50 to 250, in particular 60 to 200, preferably 80 to 180, °C.

## Revendications

1. Composés de formule 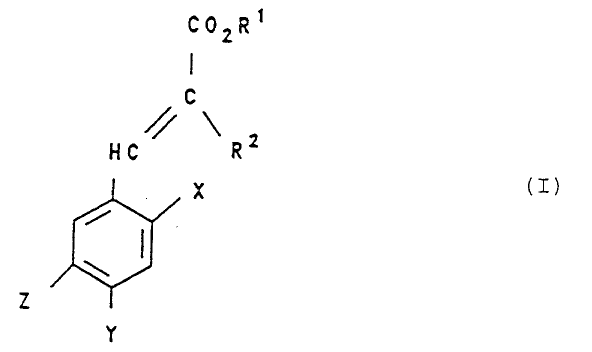 dans laquelle R¹ et R² sont identiques ou différents et représentent l'hydrogène ou un reste alkyle avec 1 à 18 atomes de carbone éventuellement contenant l'oxygène, l'azote ou un halogène, les restes X, Y et Z sont identiques et représentent un atome de fluor, de brome ou d'iode, ou dans le cas où deux des restes X, Y et Z sont identiques ou tous les restes X, Y, Z sont différents l'un de l'autre, X, Y et Z représentent un atome de fluor, de chlore, de brome ou d'iode.

2. Composés selon la revendication 1, caractérisés en ce que R² représente l'hydrogène ou un groupe méthyle.

3. Composés selon une ou plusieurs des revendications 1 et 2, caractérisés en ce que R¹ représente un reste alkyle avec 1 à 18, plus particulièrement 1 à 12 atomes de carbone, linéaire ou ramifié, éventuellement substitué par un groupe alcoxy ou un groupe acyloxy.

4. Composés selon une ou plusieurs des revendications 1 à 3, caractérisés en ce que R¹ représente un groupe -(CH₂)ₙ-OR³, où n = 2 à 6 et R³ est un reste alkyle avec 1 à 4 atomes de carbone.

5. Composés selon une ou plusieurs des revendications 1 à 4, caractérisés en ce que X et Y représentent chacun un atome de brome et Z un atome de fluor.

6. Composés selon une ou plusieurs des revendications 1 à 4, caractérisés en ce que X est un atome de brome, Y et Z chacun un atome de fluor ou Y est un atome de chlore et Z un atome de fluor et X un atome de chlore, Y un atome de brome et Z un atome de fluor, ou X et Z un atome de fluor et Y un atome de brome ou un atome de chlore.

7. Composés selon la revendication 1, caractérisés en ce qu'ils répondent à la formule 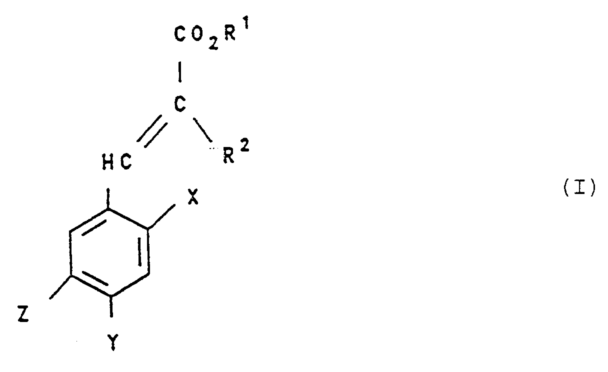 dans laquelle R¹ et R², identiques ou différents, représentent l'hydrogène ou un reste alkyle avec 1 à 18 atomes de carbone éventuellement contenant l'oxygène, l'azote ou un halogène, X représente un atome de chlore et Y et Z chacun un atome de fluor.

8. Composés selon la revendication 1, caractérisés en ce qu'ils répondent à la formule 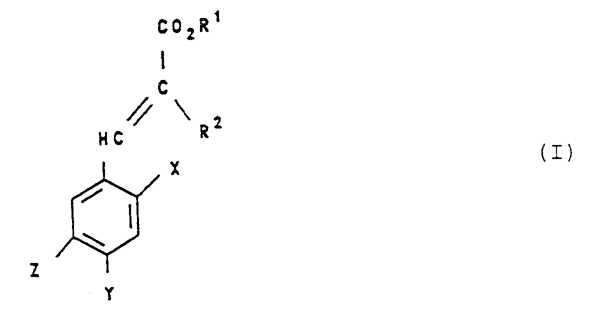 dans laquelle R¹ et R² sont identiques ou différents et représentent l'hydrogène ou un reste alkyle avec 1 à 18 atomes de carbone, éventuellement contenant l'oxygène, l'azote ou un halogène, et X, Y et Z représentent chacun un atome de fluor.

9. Composés selon la revendication 1, caractérisés en ce qu'ils répondent à la formule 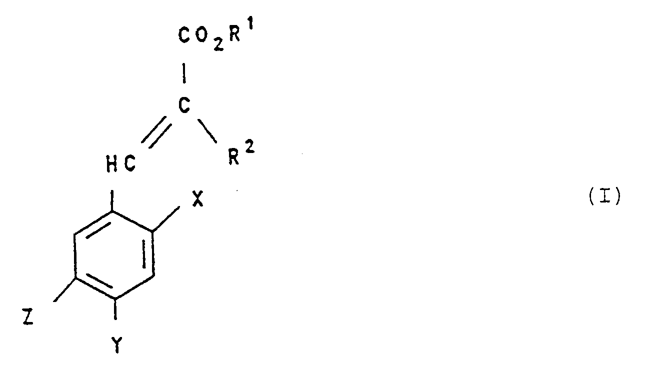 dans laquelle R¹ et R² sont identiques ou différents et représentent l'hydrogène ou un reste alkyle avec 1 à 18 atomes de carbone éventuellement contenant l'oxygène, l'azote ou un halogène, et X et Y représentent chacun un atome de chlore et Z un atome de fluor.

10. Composés selon l'une des revendications 7 à 9, caractérisés en ce que R² représente l'hydrogène ou un groupe méthyle.

11. Composés selon l'une des revendications 7 à 10, caractérisés en ce que R¹ représente un reste alkyle avec 1 à 18, plus particulièrement 1 à 12 atomes de carbone, linéaire ou ramifié, éventuellement substitué par un groupe acyloxy ou un groupe alcoxy.

12. Composés selon une ou plusieurs des revendications 7 à 11, caractérisés en ce que R¹ représente un groupe -(CH₂)ₙ-OR³, où n = 2 à 6, et R³ est un reste alkyle avec 1 à 4 atomes de carbone.

13. Composés d'aryldiazonium de formule 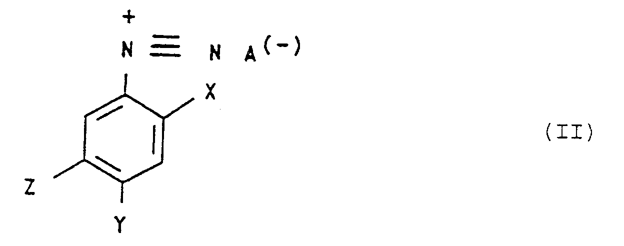 dans laquelle deux des radicaux X, Y ou Z sont identiques et représentent le fluor ou le chlore et le reste restant est le chlore ou le fluor, ou tous les restes X, Y, Z sont différents les uns des autres et X, Y et Z représentent un atome de fluor, de chlore, de brome ou d'iode et A⁽⁻⁾ un anion d'un acide ayant une valeur de pKₐ <7.

14. Composés d'aryldiazonium selon la revendication 13, caractérisés en ce qu'ils répondent à la formule 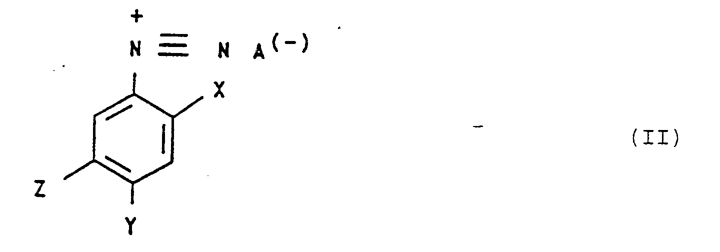 dans laquelle X est un atome de chlore et Y et Z chacun un atome de fluor.

15. Composés d'aryldiazonium selon la revendication 13, caractérisés en ce qu'ils répondent à la formule 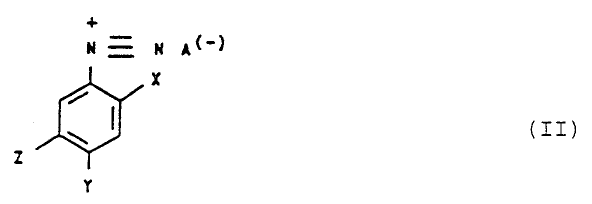 dans laquelle X et Y représentent chacun un atome de chlore et Z un atome de fluor.

16. Procédé de préparation des composés selon une ou plusieurs des revendications 1 à 12, caractérisé en ce que l'on fait réagir un sel d'aryldiazonium de formule 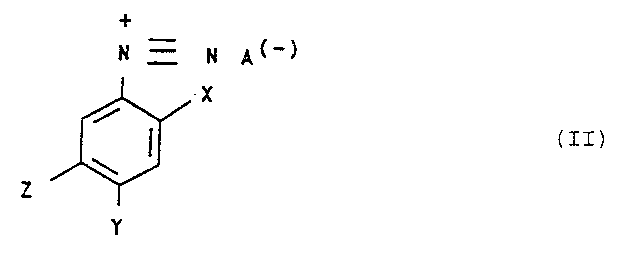 dans laquelle les restes X, Y et Z sont identiques et représentent un atome de fluor, de brome ou d'iode, dans le cas ou deux des restes X, Y et Z sont identiques ou tous les restes X, Y, Z sont différents les uns des autres, X, Y et Z sont un atome de fluor, de chlore, de brome ou d'iode et A⁽⁻⁾ un anion d'un acide ayant une valeur de pKₐ < 7, avec un composé de formule 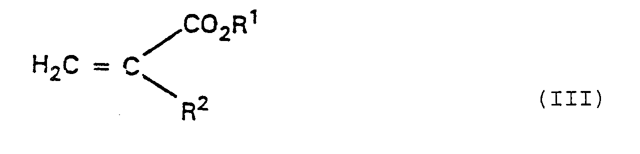 dans laquelle R¹ et R² sont identiques ou différents et représentent l'hydrogène ou un reste alkyle avec 1 à 18 atomes de carbone, éventuellement contenant l'oxygène, l'azote ou un halogène, en présence d'un catalyseur contenant du palladium, éventuellement en ajoutant une base.

17. Procédé selon la revendication 16, caractérisé en ce que l'on met en oeuvre la réaction en présence d'un solvant.

18. Procédé selon une ou plusieurs des revendications 16 et 17, caractérisé en ce que l'on utilise en tant que solvant un solvant aprotique dipolaire ou un solvant protique.

19. Procédé selon une ou plusieurs des revendications 16 à 18, caractérisé en ce que l'on met en oeuvre la réaction à une température de -20 à 120, plus particulièrement de -10 à 100, de manière préférée de 0 à 80 °C.

20. Procédé selon une ou plusieurs des revendications 16 à 19, caractérisé en ce que l'on met en oeuvre la réaction en présence d'un catalyseur supporté contenant du palladium.

21. Procédé de préparation des composés selon une ou plusieurs des revendications 1 à 12, caractérisé en ce que l'on fait réagir un halogénure d'aryle de formule 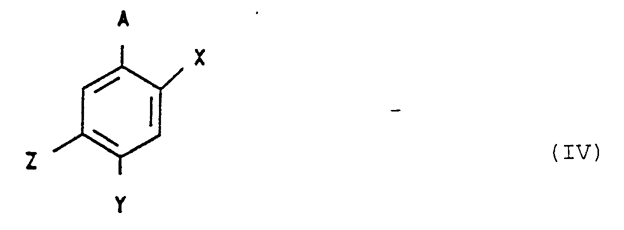 dans laquelle A est un atome de brome, X et/ou Y un atome de fluor ou de chlore et Z un atome de fluor ou A est un atome d'iode, X et/ou Y un atome de fluor, de chlore ou de brome et Z est un atome de fluor ou A est un atome de chlore, de brome ou d'iode et X, Y et Z chacun un atome de fluor, avec un composé de formule 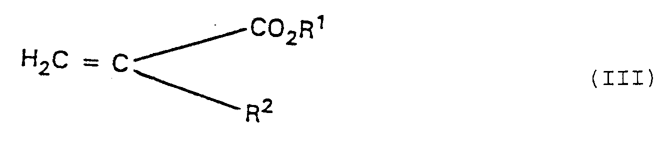 dans laquelle R¹ et R² sont identiques ou différents et représentent l'hydrogène ou un reste alkyle avec 1 à 18 atomes de carbone, éventuellement contenant l'oxygène, l'azote ou un halogène, en présence d'un catalyseur contenant du palladium, éventuellement en ajoutant une base.

22. Procédé selon la revendication 21, caractérisé en ce que l'on met en oeuvre la réaction en présence d'un solvant.

23. Procédé selon une ou plusieurs des revendications 21 et 22, caractérisé en ce que l'on utilise en tant que solvant un solvant aprotique dipolaire.

24. Procédé selon une ou plusieurs des revendications 21 à 23, caractérisé en ce que l'on met en oeuvre la réaction à une température de 50 à 250, plus particulièrement de 60 à 200, de manière préférée de 80 à 180 °C.
